# EUROPEAN PATENT SPECIFICATION

(11) **EP 1 946 235 B1**
(45) Date of publication and mention of the grant of the patent: **12.09.2018**
(21) Application number: 06821261.2
(22) Date of filing: 30.10.2006
(51) Int. Cl.: G06F 19/00

(54) **SYSTEM AND METHOD FOR CLINICAL WORKFLOW MANAGEMENT**
SYSTEM UND VERFAHREN FÜR KLINISCHE ARBEITSFLUSSVERWALTUNG
SYSTÈME ET PROCÉDÉ DE GESTION DES PROCESSUS CLINIQUES ET DE DÉCISION

(30) Priority: 31.10.2005 US 731820 P; 27.02.2006 US 777080 P
(43) Date of publication of application: 23.07.2008
(73) Proprietor: Koninklijke Philips N.V., 5656 AE Eindhoven (NL); Philips Intellectual Property & Standards GmbH, 20099 Hamburg (DE)
(72) Inventor: WEIDENHAUPT, Klaus, Briarcliff Manor, New York 10510-8001 (US); LAGOR, Charles, Briarcliff Manor, New York 10510-8001 (US)
(74) Representative: de Haan, Poul Erik
(86) International application number: PCT/IB2006/054020
(87) International publication number: WO 2007/052213

(56) References cited:
- US-A1- 2003 125 985
- US-A1- 2004 189 718

## Description

The present embodiments relate generally to medical systems and more particularly, to a clinical workflow management and decision support system and method. In the field of healthcare, various software products are commercially available, for example, hospital information systems, programs to manage workflows, statistical programs to analyze quality outcomes, and programs to develop decision support applications. No program is believed to exist that reconciles different applications into a single application, and in particular, one targeting a specific clinical problem.
Studies have shown that physicians may agree poorly when interpreting the same set of clinical information. In addition, physicians are not always up-to-date on the most recent evidence-based disease management, in particular, if the management is only partially related to their main skills. Furthermore, nurses sometimes forget treatment steps or they may perform incorrect steps, for example, giving the wrong medication or drug to a patient.

As a result, the health care that patients receive is subject to a lot of variation and the numbers of medical errors occurring through negligence are high. It has been recognized that standardizing the clinical workflow and continuously measuring the quality of the health care will reduce the number of medical errors. Thus it would be desirable to have a system and method designed to help clinicians adhere to a standard practice of medicine and to prevent medical errors by providing suggestions to the clinicians. Accordingly, an improved system and method for overcoming the problems in the art is desired.

US 2003/0125985 discloses a system for automatically ensuring adherence to clinical guidelines during the course of patient treatments.

US 2004/0189718 discloses a graphical user interface (GUI) for interacting with a user during a workflow process.
Figure 1 is a block diagram view of a clinical workflow management and decision support system according to an embodiment of the present disclosure;
Figure 2 is a screenshot view illustrating various regions of a graphical user interface of the clinical workflow management and decision support system and method according to an embodiment of the present disclosure;
Figure 3 is a screenshot view illustrating various regions of a graphical user interface of the clinical workflow management and decision support system and method according to another embodiment of the present disclosure; and
Figure 4 is a screenshot view illustrating various regions of a graphical user interface of the clinical workflow management and decision support system and method according to yet another embodiment of the present disclosure.

In the figures, like reference numerals refer to like elements. In addition, it is to be noted that the figures may not be drawn to scale.

The clinical workflow management and decision making system and method according to the embodiments of the present disclosure are designed to help clinicians adhere to a standard practice of medicine and to assist in the prevention of medical errors by providing suggestions to clinicians.

In one embodiment, a computer program stored on a computer readable media comprises a set of instructions for execution by a computer for reconciling clinical information from a hospital information system relevant to the management of a particular disease, such as stroke. It should be noted that stroke is used herein as an example for illustrative purposes only and that the embodiments of the present disclosure are not so limited. The program continuously analyzes the clinical data (i) to examine whether clinical actions adhere a predetermined protocol, (ii) to provide different healthcare professionals (e.g., physicians, nurses, CT technicians and so on) with an overview of the tasks that they have to perform, (iii) to assist healthcare professionals in making diagnostic and therapeutic decisions.

Features of the embodiments of the present disclosure include:
1.) Displaying an ideal clinical workflow and the actual steps that have been completed. This feature also calculates the time that is left until a certain target has been reached, for example, thrombolysis within three (3) hours from symptom onset.
2.) Displaying a log of clinical actions and findings that are pertinent to a particular clinical problem.
3.) Displaying a list of tasks that need to be completed.
4.) Displaying probabilities for different diseases.
5.) Providing explanations for any suggestion or suggested next step made.

Although a portion of the above-identified information may be available in separate stand-alone applications, it is believed that no system is available that combines and reconciles this complete set of clinical information in an integrated and novel manner as described herein in greater detail.

### Overall system architecture

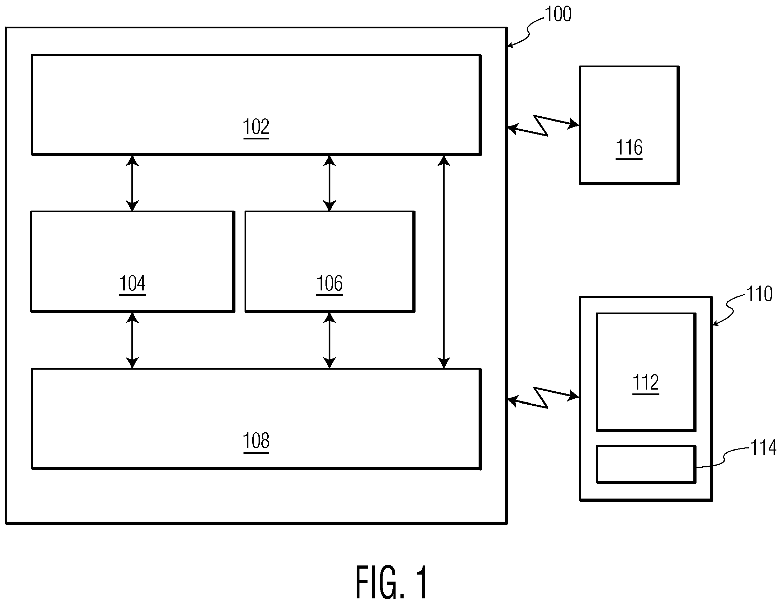

Figure 1 is a block diagram view of a clinical workflow management and decision support system configured for use within a healthcare IT infrastructure (100) according to an embodiment of the present disclosure. As illustrated in Figure 1, it is assumed that the application (102) according to the embodiments of the present disclosure is interfaced with the healthcare IT infrastructure (100) and coupled to various other systems. The various other systems can comprise, for example, a workflow/guideline management system (104), a decision support module (106), and an electronic patient record system (108). The workflow/guideline management system (104) provides a computer-interpretable representation of the clinical guideline to be followed and maintains the current state of the clinical course of actions. The decision support module (106) is configured for calculating the probabilities for a set of differential diagnoses based on the current set of evidences, corresponding to patient data. The decision support module (106) is also configured for calculating suggestions for diagnostic and therapeutic steps to be taken. In addition, the electronic patient record system (108) is configured to provide access to clinical data for the patient under consideration. In addition, while the illustration of Figure 1 is representative of one embodiment, it should be noted that the clinical workflow management and decision support system can also be independent of any existing hospital information system or electronic patient record.

According to one embodiment of the present disclosure, a method of clinical workflow management and decision making comprises receiving clinical information from a hospital information system (100), combining and reconciling the clinical information (for example, via application (102)) for use during the management of the particular clinical problem of the patient under consideration, and providing access to the combined and reconciled clinical information via a graphical user interface that comprises a plurality of interactive display regions.

The clinical information can include a variety of information relevant to the management of a particular clinical problem of a patient under consideration. Receiving clinical information includes one or more of (i) receiving clinical information from one or more source internal to the hospital information system and (ii) receiving clinical information from one or more source external to the hospital information system. For example, receiving from a source internal to the hospital information system 100 can include receiving clinical information from one or more of (a) a workflow/guideline management system (104), (b) a decision support module (106), (c) an electronic patient record system (108), and (d) a system user terminal (110) coupled to the hospital information system. The system user terminal (110) can include for example, a tablet PC or other suitable electronic device having a display (112) and an input (114). It should be noted that the system user terminal can comprise one or more of a computer, a personal digital assistant, a cellular telephone, a mobile communications device, or similar device, such as an electronic device wherein the display also serves as an input device. In addition, receiving from a source external to the hospital information system (100) can include, for example, receiving clinical information from a remote device (116), wherein the remote device can be configured for being suitably coupled to the hospital information system and for communicating clinical information to the hospital information system. A remote device (116) can comprise, for example, one or more of a computer, a personal digital assistant, a cellular telephone, a mobile communications device, or similar device.

In one embodiment, the combining and reconciling comprises repeatedly analyzing the clinical information for: (a) determining an overview of clinical actions as a function of the clinical information, (b) examining whether clinical actions adhere to a predetermined protocol, and (c) determining suggested next step information suitable for use in assisting with diagnostic and therapeutic decisions to be made by one or more healthcare providers in conjunction with treatment of the particular clinical problem. The clinical actions can comprise, for example, one or more of the following: (a) clinical actions performed, (b) clinical actions currently being performed, and (c) clinical actions yet to be performed. In addition, the one or more healthcare providers include at least one selected from the group consisting of a doctor, nurse, healthcare professional, and healthcare assistant.

In one embodiment, the predetermined protocol includes (i) one or more ideal clinical actions and (ii) timing for an occurrence of the one or more ideal clinical actions as a function of the clinical information and the particular clinical problem. In addition, the predetermined protocol comprises an evidence based protocol. Furthermore, repeatedly analyzing the clinical information can include continuously analyzing the clinical information throughout at least a critical portion of treatment of the particular clinical problem.

### Description of the GUI

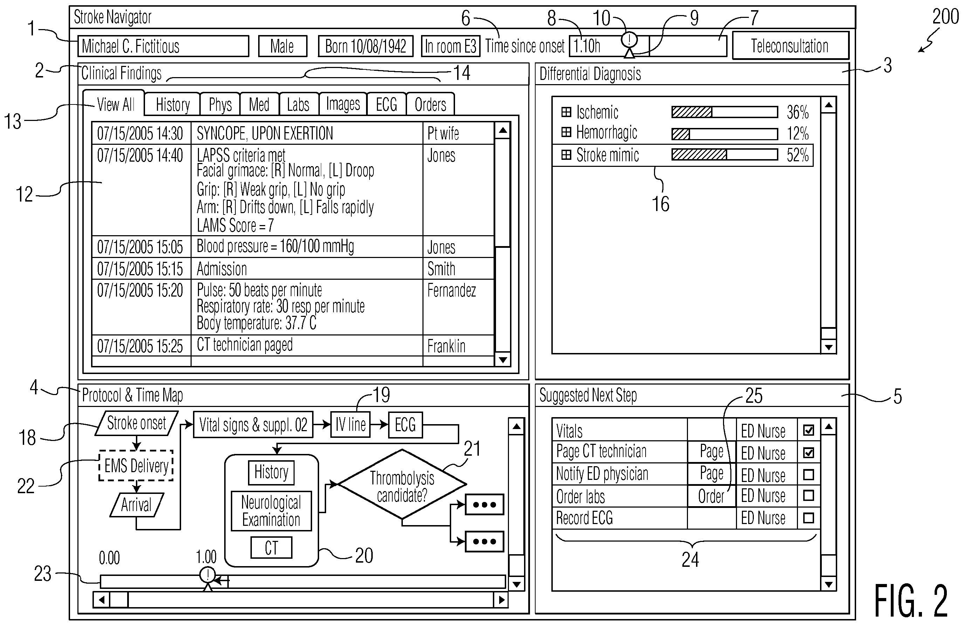

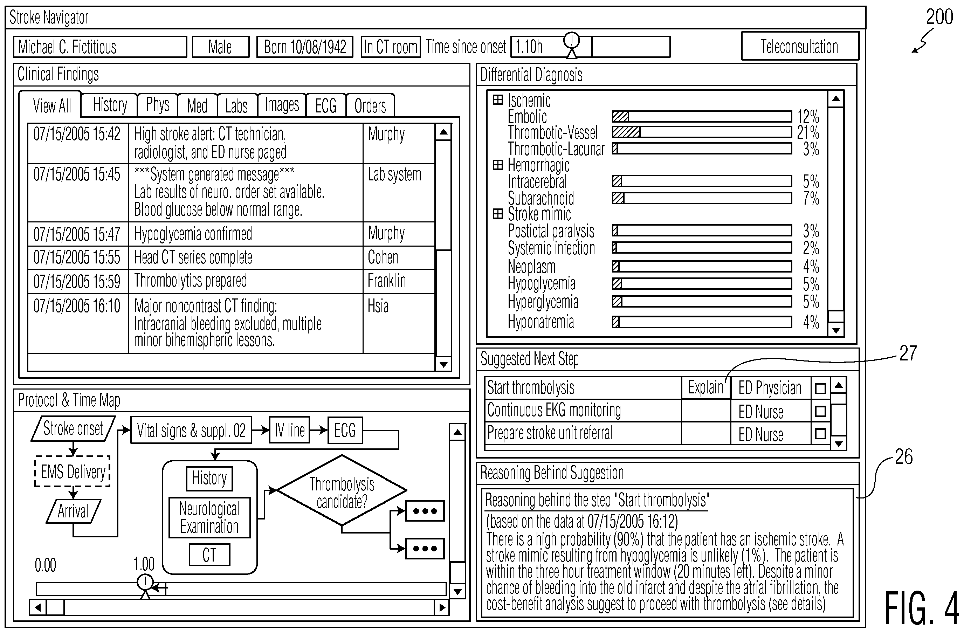

As discussed above, the method of clinical workflow management and decision making provides access to combined and reconciled clinical information via a graphical user interface (GUI). The graphical user interface includes a plurality of interactive display regions, as will be discussed below. Figure 2 is a screenshot view (200) illustrating the various regions of the graphical user interface of the clinical workflow management and decision support system and method according to one embodiment of the present disclosure. In particular, Figure 2 illustrates the GUI of the combining and reconciling of clinical information application as applied in the management of stroke. As will be discussed, the graphical user interface (200) is subdivided in various sections comprising: a high-level overview row (1) for displaying the most important patient data; a clinical findings window (2); a differential diagnosis window (3); a protocol and time map window (4); a suggested next step window (5); and an explanation window (26) as is shown in Figure 4. Stated in a slightly different manner, the graphical user interface (200) comprises a plurality of interactive display regions. The interactive display regions include a high-level patient data overview region (1), a clinical findings region (2), a differential diagnosis region (3), a protocol and time map region (4), and a suggested next step region (5).

### High-level overview row

The top row (1) condenses the most important information about the patient under consideration at a glance: the name of the patient, his/her gender, birthday, and current location. In other words, the high-level patient data overview region (1) is configured for providing important information about the patient under consideration. The important information includes, for example, the patient's name, gender, information from which the patient's age can be discerned, and the patient's current physical location. The high-level patient data overview region is further configured for providing a graphical bar representation (6) of a time since onset of an event giving rise to the particular clinical problem of the patient under consideration.

### "Time since onset"- Bar

The "Time since onset" bar (6) is of major importance for diseases that are characterized by a narrow time window between the onset of the symptoms and the start of the treatment. It provides a high-level overview of the time passed since symptom onset and of the progress within the protocol. The entire bar (7) represents the full time window (e.g. 3 hours in the case of stroke). A portion of the bar indicated by reference numeral (8) can be displayed as a colored fraction that represents the time already gone since symptom onset. As shown for illustration purposes only, the time since onset in this example is 1:10 hours.

The combining and reconciling of clinical information application (102) maintains a model of a normative workflow in terms of the medical actions to be performed and the time needed for those steps. It also maintains a model of the actual course of actions. The application is, thus, able to compare the actual course of actions with the normative workflow and can determine whether or not there is a delay. In order to visualize the delay, a triangle (9) indicates the nominal position of the current step according to an ideal workflow. In this scenario, it is assumed that there is a delay, so the current step is placed before the actual time, thereby indicating that it ideally should have happened already 10 minutes before. To strengthen the sense of urgency even more, there is an additional exclamation mark (10) and the color of the bar (8) can be changed, for example, to the color orange. If the current handling of the patient was in time (or even ahead), the bar (8) would be green and the triangle (9) would have been placed at the end of the colored portion of the bar (or even more to the right). If the handling was even later, the bar (8) would turn red and the triangle (9) would have been placed more to the left.

### "Clinical findings" window

The clinical findings window or region (2) is configured for providing a log of clinical findings, events, and actions pertinent to the particular clinical problem. The clinical findings, events, and actions are based upon the clinical information. In one embodiment, each row of the log of clinical findings, events, and actions provides an indication of a date and time, a description, results, and an identification of a party that performed a corresponding one of the clinical findings, events, and actions. In addition, entries within the log of clinical findings, events, and actions can be internally tagged by definable categories. Tagging by definable categories enables filtering of the entries using a corresponding tab for a desired one of the categories. Furthermore, the definable categories can include one or more of history, physical, medical, laboratory, images, ECGs, orders, or other suitable categories according to the requirements of the particular clinical application.

In our illustrative example, the top left window (2) in Figure 2 comprises the log of clinical findings, events, and actions that have taken place. The data displayed may be retrieved from a hospital information system or directly entered by the user into the application. Each row (12) indicates the date/time of the clinical action, a detailed description of the action and its results, and the user performing the action. The individual entries are internally tagged by definable categories (for example, History, Phys, Med, Lab, Images, ECGs, Orders, etc.), so it is possible to either view them all using the "View all" tab (13) or to filter them using the corresponding tabs (14) for the individual categories.

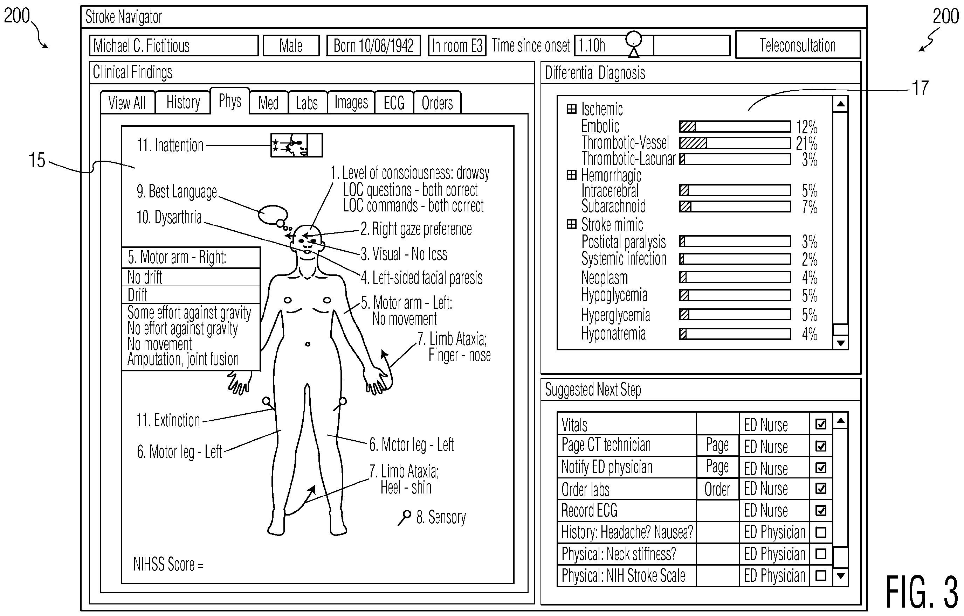

Depending on the nature of the data displayed in the clinical findings window (2), it should be noted that customized data display and entry forms may also be used. This is illustrated in Figure 3, where a special form (15) for entering and displaying the results of a physical examination is shown.

### "Differential diagnosis" window

The differential diagnosis window or region (3) is configured for providing a representation of probabilities for one or more differential diagnoses as a function of currently available clinical information. In one embodiment, the differential diagnosis region lists possible diagnoses vertically, wherein each diagnosis is identified by its name and a bar having a length representative of a current probability of the corresponding diagnosis. In addition, a diagnosis may be structured hierarchically into a set of sub-diagnoses. In such an instance, the diagnosis with the set of sub-diagnoses can be graphically accounted for by one of expanding or collapsing a diagnosis in a tree-view like manner.

In our illustrative example, it is assumed that the application (102) is coupled to the decision support module (106) that can calculate the probabilities for a set of differential diagnoses, given the currently available set of evidences (i.e., clinical findings). In the embodiment considered here, the decision support module (106) is based on a Bayesian network model that captures the causal relationships between evidences (i.e., results of clinical tests) and various stroke types, stroke mimics and a number of underlying pathologies.

The differential diagnosis window (3) lists all possible diagnoses vertically. Each possible diagnosis is indicated by its name and a bar representing its current probability (16). A diagnosis may be structured hierarchically into a set of sub-diagnoses. This is graphically accounted for by the possibility to expand and collapse a diagnosis in a tree-view like manner (17).

As new evidence becomes available, i.e. clinical findings are added or updated, the application (102) queries the decision support module (106) in order to update the probabilities for the various differential diagnoses. This results in a change of the corresponding probability bars in the differential diagnosis window (3).

### "Protocol and time map" window

The protocol and time map window or region (4) is configured for providing a graphical representation of a clinical workflow representative of the predetermined protocol, wherein the clinical workflow has been reconciled as a function of the overview of clinical actions. The protocol and time map region is further configured for providing a graphical representation of a clinical workflow time map. The clinical workflow time map includes, for example, a correlation between a progression of the clinical workflow with (a) a time that has elapsed from a symptom onset of the particular clinical problem and (b) a time that is remaining within a period of time for treatment of the particular clinical problem. In addition, the protocol and time map region highlights an adherence to time constraints of the clinical workflow by horizontally aligning one or more individual steps of the clinical workflow with a corresponding time on the time map. In one embodiment, the time map is configured to begin with a time corresponding to the symptom onset. Furthermore, the protocol and time map region also provides visualization, in connection with the time map, of a deviation of a current step from its ideal timing in the clinical workflow.

In our illustrative example, the information shown in window (4) is retrieved by the application (102) from the connected workflow or guideline management system (104). The window (4) displays the ideal clinical workflow and the actual steps that have been completed. In the graphical representation, the following graphical shapes connected by arcs are used to distinguish different types of steps:
Rhombus (18) for events;
Rectangle (19) for an action;
Rounded rectangle for compound steps (without a predefined sequence among the sub-steps) (20); and
Diamonds for decision steps (21).

Parts of the map can be inflated into a single shape to save screen space. This is indicated by using a dashed line (e.g. in the step "EMS Delivery" that actually comprises several steps) (22). The protocol map further uses different colors for steps that have already been completed (e.g., blue), are currently active (e.g., yellow) and are not yet performed (e.g., white).

According to one embodiment of the present disclosure, one element is to correlate the progression within the clinical workflow with the time that has passed by or elapsed since symptom onset and the time that is left until the end of the treatment window, respectively. To highlight the adherence to time constraints, the individual steps are aligned horizontally with a time line (23) that starts with the symptom onset. The deviation of the current step from its timing in an ideal workflow is visualized in the timeline, similarly, as described above with respect to the "Time since onset" bar (6) of window (1). In one embodiment, the time bars (6) and (23) are substantially the same or identical.

The application (102) can also calculate the estimated time needed for the remaining steps to be done. To do this, it takes into account information about the typical duration of a step and the current availability of medical staff and resources. These durations may be specified in terms of time ranges, so it is possible to calculate a best case, worst case, and average case estimation. Depending on whether the remaining time until a certain target (e.g. the end of a time window for the thrombolysis therapy) is less than the time required for the remaining steps, the application (102) issues a warning, combined with the suggestion to put certain steps on a fast track (e.g. to prioritize the CT scan for this patient) or even to bypass certain steps.

### "Suggested next steps" window

The suggested next step window or region (5) is configured for providing the suggested next step information having been determined to assist with diagnostic and therapeutic decisions to be made by one or more healthcare providers. In another embodiment, the suggested next step information can comprise (a) a suggested next action derived from a current state of execution of the clinical workflow and/or (b) a suggested next action resulting from a query to a decision support module in connection with a differential diagnosis of the differential diagnosis region. In the latter instance, the query is configured for finding one or more suitable diagnostic steps to discriminate between multiple differential diagnoses.

In addition, the suggested next step information can be presented in one or more rows. In one embodiment, the one or more rows are characterized by a description of an action of a corresponding next step, identification of a party responsible for performing the action of the corresponding next step, and a check mark box. The occurrence of a check mark within the check mark box indicates that the corresponding next step has been completed. In another embodiment, the suggested next step information can further include an optional button that, in response to being selected, executes a corresponding action of the suggested next step automatically.

In our illustrative example, the suggested next step window (5) shows a list of steps suggested by the system. The suggestions are on the one hand derived from the current state of the guideline execution. On the other hand, the physician may query the decision support module 106 in the "Differential diagnosis" window (3) to find the best diagnostic steps to discriminate between multiple differential diagnoses. The result of such a query can also listed in the "Suggested next steps" window.

Each step (24) in the suggested next steps window (5) is characterized by a short description, the actor responsible for performing the action, and a check mark, that indicates whether the step has been completed. For each step, there may be an optional button, which executes the corresponding action automatically (e.g., paging the CT technician) (25).

### "Explanation" window

In another embodiment, the graphical user interface further comprises an explanation window or region (26) shown in Figure 4. The explanation region is configured for providing an explanation for a given suggested next step. The explanation can include, for example, a description of the reasoning behind the given suggested next step derived from an underlying decision support module. In addition, the explanation within the explanation region can be provided in response to activation via an explain button, wherein the explain button is provided with the corresponding suggested next step information within the suggested next step region.

In the explanation window (26), a detailed description of the reasoning behind a suggested step is displayed. This information is derived from the underlying decision support module (106). Furthermore, the explanation window (26) can be activated, for example, using the "Explain" button (27) in the "Suggested next step" window (5).

### Medical Applications

The embodiments of the present disclosure will generally help in the management of acute diseases in a complex clinical setting. This in particular holds for diseases where adherence to normative guidelines is crucial and which are characterized by complex decision paths. Prime examples are the management of patients with trauma, myocardial infarction, or stroke. One application for the embodiments of the present disclosure includes the area of managing acute stroke patients.

In addition to the above, the embodiments of the present disclosure also include computer software or a computer program product. The computer program product includes a computer readable media having a set of instructions executable by a computer for carrying out the methods of clinical workflow management and decision making as described and discussed herein. The computer readable media can include any suitable computer readable media for a given clinical treatment system application. Still further, the computer readable media may also include a network communication media. Examples of network communication media include, for example, an intranet, the Internet, or an extranet. In one embodiment, the healthcare IT infrastructure can comprise a computer, network, or other suitable computer based system.

According to another embodiment, a clinical workflow management and decision making system comprises an input for receiving clinical information from a hospital information system, a processor (or suitable computer system) for combining and reconciling the clinical information for use during the management of the particular clinical problem of the patient under consideration, and a graphical user interface for providing access to the combined and reconciled clinical information, wherein the graphical user interface comprises a plurality of interactive display regions. The clinical information includes a variety of information relevant to management of a particular clinical problem of a patient under consideration. In addition, the combining and reconciling comprises repeatedly analyzing the clinical information for: (a) determining an overview of clinical actions as a function of the clinical information, (b) examining whether clinical actions adhere to a predetermined protocol. In one embodiment, the predetermined protocol includes (i) one or more ideal clinical actions and (ii) timing for an occurrence of the one or more ideal clinical actions as a function of the clinical information and the particular clinical problem. The combining and reconciling further comprises (c) determining suggested next step information suitable for use in assisting with diagnostic and therapeutic decisions to be made by one or more healthcare providers in conjunction with treatment of the particular clinical problem.

Further with respect to the clinical workflow management and decision making system, the graphical user interface comprises a plurality of interactive display regions that include at least (i) a protocol and time map region, (ii) a clinical findings region, (iii) a differential diagnosis region, (iv) a suggested next step region, and (v) a high-level patient data overview region. The graphical user interface may further comprise an explanation region. Similar as discussed herein above, the protocol and time map region is configured for providing a graphical representation of a clinical workflow representative of the predetermined protocol, wherein the clinical workflow has been reconciled as a function of the overview of clinical actions. The protocol and time map region is further configured for providing a graphical representation of a clinical workflow time map. In one embodiment, the time map includes a correlation between a progression of the clinical workflow with (a) a time that has elapsed from a symptom onset of the particular clinical problem and (b) a time that is remaining within a period of time for treatment of the particular clinical problem.

Still further with respect to the clinical workflow management and decision making system, and also similar as discussed herein above, the clinical findings region is configured for providing a log of clinical findings, events, and actions pertinent to the particular clinical problem, wherein the clinical findings, events, and actions are based upon the clinical information. In addition, the differential diagnosis region is configured for providing a representation of probabilities for one or more differential diagnoses as a function of currently available clinical information. The suggested next step region is configured for providing the suggested next step information having been determined to assist with diagnostic and therapeutic decisions to be made by one or more healthcare providers. Furthermore, the high-level patient data overview region is configured for providing important information about the patient under consideration, wherein the important information includes the patient's name, gender, information from which the patient's age can be discerned, and the patient's current physical location. The high-level patient data overview region is further configured for providing a graphical bar representation of a time since onset of an event giving rise to the particular clinical problem of the patient under consideration.

Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure can be applied to trauma, myocardial infarction, or stroke. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A computer-implemented method of clinical workflow management and decision making comprising:
receiving clinical information from a hospital information system (100), the clinical information including information relevant to management of a particular clinical problem of a patient under consideration;
repeatedly:
(a) determining an overview of clinical actions as a function of the clinical information, the clinical actions comprising at least one selected from the group consisting of: clinical actions performed, clinical actions currently being performed, and clinical actions not yet performed,
(b) examining whether the clinical actions adhere to a predetermined protocol, the predetermined protocol including
(i) one or more ideal clinical actions and
(ii) a timing for an occurrence of the one or more ideal clinical actions as a function of the clinical information and the particular clinical problem, and
(c) determining suggested next step information suitable for use in assisting with diagnostic and therapeutic decisions to be made by one or more healthcare providers in conjunction with treatment of the particular clinical problem, and
displaying the suggested next step information via a graphical user interface (200) that comprises a plurality of interactive display regions (5), wherein the plurality of interactive display regions comprise:
a differential diagnosis region for providing a representation of probabilities for one or more differential diagnoses as a function of currently available clinical information, and
a suggested next step region for providing the suggested next step information having been determined to assist with diagnostic and therapeutic decisions to be made by one or more healthcare providers, wherein the suggested next step information comprises one selected from the group consisting of (a) a suggested next action derived from a current state of execution of the clinical workflow and currently available clinical information, and (b) a suggested next action resulting from a query to a decision support module in connection with a differential diagnosis of the differential diagnosis region, the query configured for finding one or more suitable diagnostic steps to discriminate between multiple differential diagnoses..

2. The method of claim 1, wherein the receiving clinical information includes receiving from one or more source internal to the hospital information system; and wherein receiving from one or more source internal to the hospital information system includes receiving clinical information from one selected from the group consisting of (a) a workflow/guideline management system, (b) a decision support module, (c) an electronic patient record system, and (d) a system user device.

3. The method of claim 1, wherein the receiving clinical information includes receiving from one or more source external to the hospital information system; and wherein receiving from one or more source external to the hospital information system includes receiving clinical information from a remote device.

4. The method of claim 1, wherein the one or more healthcare providers include at least one selected from the group consisting of a doctor, nurse, healthcare professional, and healthcare assistant.

5. A computer readable media having stored thereon a computer program, wherein said program comprises a set of instructions that, when executed by a computer, carry out the steps of the method of claim 1.

6. A clinical workflow management and decision making system comprising:
means for receiving clinical information from a hospital information system (100), the clinical information including information relevant to management of a particular clinical problem of a patient under consideration;
means for repeatedly:
(a) determining an overview of clinical actions as a function of the clinical information, the clinical actions comprising at least one selected from the group consisting of: clinical actions performed, clinical actions currently being performed, and clinical actions not yet performed,
(b) examining whether the clinical actions adhere to a predetermined protocol, the predetermined protocol including
(i) one or more ideal clinical actions and
(ii) a timing for an occurrence of the one or more ideal clinical actions as a function of the clinical information and the particular clinical problem, and
(c) determining suggested next step information suitable for use in assisting with diagnostic and therapeutic decisions to be made by one or more healthcare providers in conjunction with treatment of the particular clinical problem, and
means for displaying the suggested next step information via a graphical user interface (200) that comprises a plurality of interactive display regions (5), wherein the plurality of interactive display regions comprise:
a differential diagnosis region for providing a representation of probabilities for one or more differential diagnoses as a function of currently available clinical information, and
a suggested next step region for providing the suggested next step information having been determined to assist with diagnostic and therapeutic decisions to be made by one or more healthcare providers, wherein the suggested next step information comprises one selected from the group consisting of (a) a suggested next action derived from a current state of execution of the clinical workflow and currently available clinical information, and (b) a suggested next action resulting from a query to a decision support module in connection with a differential diagnosis of the differential diagnosis region, the query configured for finding one or more suitable diagnostic steps to discriminate between multiple differential diagnoses.

7. The system of claim 6, wherein the plurality of interactive display regions further comprises:
(i) a protocol and time map region for providing a graphical representation of a clinical workflow representative of the predetermined protocol, wherein the protocol and time map region is further for providing a graphical representation of a clinical workflow time map, wherein the time map includes a correlation between a progression of the clinical workflow with (a) a time that has elapsed from a symptom onset of the particular clinical problem and (b) a time that is remaining within a period of time for treatment of the particular clinical problem, and
(ii) a clinical findings region for providing a log of clinical findings, events, and actions pertinent to the particular clinical problem, wherein the clinical findings, events, and actions are based upon the clinical information.

8. The system of claim 7, wherein the plurality of interactive display regions further comprise:
a high-level patient data overview region for providing information about the patient under consideration, wherein the information includes the patient's name, gender, information from which the patient's age can be discerned, and the patient's current physical location, and wherein the high-level patient data overview region is further for providing a graphical bar representation of a time since onset of an event giving rise to the particular clinical problem of the patient under consideration.

9. The system of claim 7, wherein the plurality of interactive display regions further comprise:
an explanation region for providing an explanation for a given suggested next step, the explanation including a description of a reasoning behind the given suggested next step derived from an underlying decision support module, wherein the explanation within the explanation region is provided in response to activation via an explain button, wherein the explain button is provided with the corresponding suggested next step information within the suggested next step region.

10. The system of claim 7, wherein the protocol and time map region further highlights an adherence to time constraints of the clinical workflow by horizontally aligning one or more individual steps of the clinical workflow with a corresponding time on the time map.

11. The system of claim 10, wherein the protocol and time map region further provides, in connection with the time map, a visualization of a deviation of a current step from its ideal timing in the clinical workflow.

12. The system of claim 7, wherein each row of the log of clinical findings, events, and actions provides an indication of a date and time, a description, results, and an identification of a party that performed a corresponding one of the clinical findings, events, and actions, further wherein entries within the log of clinical findings, events, and actions are internally tagged by definable categories to enable filtering of the entries using a corresponding tab for a desired one of the categories, and still further wherein the definable categories include one or more selected from the group consisting of history, physical, medical, laboratory, images, ECGs, and orders.

13. The system of claim 7, wherein the differential diagnosis region lists possible diagnoses vertically, wherein each diagnosis is identified by its name and a bar having a length representative of a current probability of the corresponding diagnosis.

14. The system of claim 13, wherein a diagnosis can further be structured hierarchically into a set of sub-diagnoses that are graphically accounted for by one of expanding or collapsing a diagnosis in a tree-view like manner.

15. The system of claim 7, wherein suggested next step information is presented in one or more rows **characterized by** a description of an action of a corresponding next step, identification of a party responsible for performing the action of the corresponding next step, and a check mark box, wherein an occurrence of a check mark within the check mark box indicates that the corresponding next step has been completed.

16. The system of claim 7, further wherein completed steps of the suggested next step information are modified in a manner to visually indicate completion of the same.

17. The system of claim 16, further comprising a button that, in response to being selected, executes a corresponding action of the suggested next step automatically.

## Patentansprüche

1. Computerimplementiertes Verfahren für klinische Arbeitsflussverwaltung und Entscheidungsfindung, umfassend:
Empfangen von klinischen Informationen von einem Krankenhausinformationssystem (100), wobei die klinischen Informationen Informationen beinhalten, welche für die Verwaltung eines bestimmten klinischen Problems eines Patienten unter Beobachtung relevant sind;
wiederholt:
(a) Bestimmen einer Übersicht von klinischen Maßnahmen als eine Funktion der klinischen Informationen, wobei die klinischen Maßnahmen zumindest eine, ausgewählt aus der Gruppe, bestehend aus: durchgeführten klinischen Maßnahmen, derzeit durchgeführten klinischen Maßnahmen und noch nicht durchgeführten klinischen Maßnahmen umfassen,
(b) Bestimmen, ob die klinischen Maßnahmen sich an ein vorbestimmtes Protokoll halten, wobei das vorbestimmte Protokoll beinhaltet
(i) eine oder mehrere ideale klinische Maßnahmen und
(ii) eine Zeitvorgabe für ein Auftreten von der einen oder den mehreren idealen klinischen Maßnahmen als eine Funktion der klinischen Informationen und des bestimmten klinischen Problems, und
(c) Bestimmen von Informationen für den vorgeschlagenen nächsten Schritt, welche zur Verwendung bei der Unterstützung bei Diagnose- und Therapieentscheidungen, welche von einem oder mehreren Gesundheitsdienstleistern in Verbindung mit Behandlung des bestimmten klinischen Problems getroffen werden sollen, geeignet sind, und
Anzeigen der Informationen für den vorgeschlagenen nächsten Schritt über eine grafische Benutzeroberfläche (200), welche eine Vielzahl von interaktiven Anzeigeregionen (5) umfasst, wobei die Vielzahl von interaktiven Anzeigeregionen umfasst:
eine Differenzialdiagnoseregion zum Bereitstellen einer Darstellung von Wahrscheinlichkeiten für eine oder mehrere Differenzialdiagnosen als eine Funktion von derzeit verfügbaren klinischen Informationen, und
eine Region für den vorgeschlagenen nächsten Schritt zum Bereitstellen der Informationen für den vorgeschlagenen nächsten Schritt, welche bestimmt wurden, um bei Diagnose- und Therapieentscheidungen, welche von einem oder mehreren Gesundheitsdienstleistern getroffen werden sollen, zu unterstützen, wobei die Informationen für den vorgeschlagenen nächsten Schritt eine, ausgewählt aus der Gruppe, bestehend aus (a) einer vorgeschlagenen nächsten Maßnahme, welche aus einem derzeitigen Untersuchungszustand des klinischen Arbeitsflusses und derzeit verfügbaren klinischen Informationen abgeleitet wurde, und (b) einer vorgeschlagenen nächsten Maßnahme, welche aus einer Abfrage eines Entscheidungsunterstützungsmoduls in Verbindung mit einer Differenzialdiagnose der Differenzialdiagnoseregion resultiert, wobei die Abfrage konfiguriert ist, eine oder mehrere passende Diagnoseschritte zur Unterscheidung zwischen mehreren Differenzialdiagnosen zu finden, umfassen.

2. Verfahren nach Anspruch 1, wobei das Empfangen von klinischen Informationen Empfangen von einer oder mehreren Quellen innerhalb des Krankenhausinformationssystems beinhaltet; und wobei Empfangen von einer oder mehreren Quellen innerhalb des Krankenhausinformationssystems Empfangen von klinischen Informationen von einem, ausgewählt aus der Gruppe, bestehend aus (a) einem Arbeitsfluss/Anweisungsverwaltungssystem, (b) einem Entscheidungsunterstützungsmodul, (c) einem elektronischen Patientendatensystem und (d) einer Systembenutzervorrichtung beinhaltet.

3. Verfahren nach Anspruch 1, wobei das Empfangen von klinischen Informationen Empfangen von einer oder mehreren Quellen außerhalb des Krankenhausinformationssystems beinhaltet; und wobei Empfangen von einer oder mehreren Quellen außerhalb des Krankenhausinformationssystem Empfangen von klinischen Informationen von einer Remote-Vorrichtung beinhaltet.

4. Verfahren nach Anspruch 1, wobei der eine oder die mehreren Gesundheitsdienstleister zumindest einen, ausgewählt aus der Gruppe, bestehend aus einem Arzt, einer Krankenschwester, einem Gesundheitsexperten und einem Gesundheitsassistenten beinhalten.

5. Computerlesbares Medium, mit einem darauf gespeicherten Computerprogramm, wobei das Programm einen Satz von Anweisungen umfasst, welche, wenn von einem Computer ausgeführt, die Schritte des Verfahrens nach Anspruch 1 durchführen.

6. Klinisches Arbeitsflussverwaltung- und Entscheidungsfindungssystem, umfassend:
Mittel zum Empfangen von klinischen Informationen von einem Krankenhausinformationssystem (100), wobei die klinischen Informationen Informationen beinhalten, welche für die Verwaltung eines bestimmten klinischen Problems eines Patienten unter Beobachtung relevant sind;
Mittel für wiederholtes:
(a) Bestimmen einer Übersicht von klinischen Maßnahmen als eine Funktion der klinischen Informationen, wobei die klinischen Maßnahmen zumindest eine, ausgewählt aus der Gruppe, bestehend aus: durchgeführten klinischen Maßnahmen, derzeit durchgeführten klinischen Maßnahmen und noch nicht durchgeführten klinischen Maßnahmen umfassen,
(b) Bestimmen, ob die klinischen Maßnahmen sich an ein vorbestimmtes Protokoll halten, wobei das vorbestimmte Protokoll beinhaltet
(i) eine oder mehrere ideale klinische Maßnahmen und
(ii) eine Zeitvorgabe für ein Auftreten von der einen oder den mehreren klinischen Maßnahmen als eine Funktion der klinischen Informationen und des bestimmten klinischen Problems, und
(c) Bestimmen von Informationen für den vorgeschlagenen nächsten Schritt, welche zur Verwendung bei der Unterstützung bei Diagnose- und Therapieentscheidungen, welche von einem oder mehreren Gesundheitsdienstleistern in Verbindung mit Behandlung des bestimmten klinischen Problems getroffen werden sollen, geeignet sind, und
Mittel zum Anzeigen der Informationen für den vorgeschlagenen nächsten Schritt über eine grafische Benutzeroberfläche (200), welche eine Vielzahl von interaktiven Anzeigeregionen (5) beinhaltet, wobei die Vielzahl von interaktiven Anzeigeregionen umfasst:
eine Differenzialdiagnoseregion zum Bereitstellen einer Darstellung von Wahrscheinlichkeiten für eine oder mehrere Differenzialdiagnosen als eine Funktion von derzeit verfügbaren klinischen Informationen, und
eine Region für den vorgeschlagenen nächsten Schritt zum Bereitstellen der Informationen für den vorgeschlagenen nächsten Schritt, welche bestimmt wurden, um bei Diagnose- und Therapieentscheidungen, welche von einem oder mehreren Gesundheitsdienstleistern getroffen werden sollen, zu unterstützen, wobei die Informationen für den vorgeschlagenen nächsten Schritt eine, ausgewählt aus der Gruppe, bestehend aus (a) einer vorgeschlagenen nächsten Maßnahme, welche aus einem derzeitigen Untersuchungszustand des klinischen Arbeitsflusses und derzeit verfügbaren klinischen Informationen abgeleitet wurde, und (b) einer vorgeschlagenen nächsten Maßnahme, welche aus einer Abfrage eines Entscheidungsunterstützungsmoduls in Verbindung mit einer Differenzialdiagnose der Differenzialdiagnoseregion resultiert, wobei die Abfrage konfiguriert ist, eine oder mehrere passende Diagnoseschritte zur Unterscheidung zwischen mehreren Differenzialdiagnosen zu finden, umfassen.

7. System nach Anspruch 6, wobei die Vielzahl von interaktiven Anzeigeregionen weiter umfasst:
(i) ein Protokoll und einen Zeitplanregion zum Bereitstellen einer grafischen Darstellung eines klinischen Arbeitsflusses, welcher für das vorbestimmte Protokoll repräsentativ ist, wobei das Protokoll und die Zeitplanregion weiter zum Bereitstellen einer grafischen Darstellung eines Zeitplans eines klinischen Arbeitsflusses dient, wobei der Zeitplan eine Korrelation zwischen einer Progression des klinischen Arbeitsflusses mit (a) einer Zeit, welche seit einem Auftreten des bestimmten klinischen Problems abgelaufen ist, und (b) einer Zeit, welche innerhalb einer Zeitspanne zur Behandlung des bestimmten klinischen Problems verbleibt, beinhaltet, und
(ii) eine klinische Erkenntnisregion zum Bereitstellen eines Protokolls von klinischen Erkenntnissen, Ereignissen und Maßnahmen, welche für das bestimmte klinische Problem zweckdienlich sind, wobei die klinischen Erkenntnisse, Ereignisse und Maßnahmen auf den klinischen Informationen basieren.

8. System nach Anspruch 7, wobei die Vielzahl von interaktiven Anzeigeregionen weiter umfassen:
eine hochrangige Patientendatenübersichtsregion zum Bereitstellen von Informationen über den Patienten unter Beobachtung, wobei die Informationen den Namen, das Geschlecht des Patienten, Informationen, aus denen das Alter des Patienten erkennbar ist, und den derzeitigen physikalischen Aufenthaltsort des Patienten beinhalten, und wobei die hochrangigen Patientendatenübersichtsregion weiter zum Bereitstellen einer grafischen Balkendarstellung einer Zeit seit dem Auftreten eines Ereignisses, welches Anlass des bestimmten klinischen Problems des Patienten unter Beobachtung war, dient.

9. System nach Anspruch 7, wobei die Vielzahl von interaktiven Anzeigeregionen weiter umfassen:
eine Erklärungsregion zum Bereitstellen einer Erklärung für einen gegebenen vorgeschlagenen nächsten Schritt, wobei die Erklärung eine Beschreibung einer Begründung hinter dem gegebenen vorgeschlagenen nächsten Schritt beinhaltet, welche von einem zugrundeliegenden Entscheidungsunterstützungsmodul abgeleitet ist, wobei die Erklärung innerhalb des Erklärungsmoduls als Reaktion auf Aktivierung über eine Erklärungsschaltfläche bereitgestellt wird, wobei die Erklärungsschaltfläche mit den zugehörigen Informationen für den vorgeschlagenen nächsten Schritt innerhalb der Region für den vorgeschlagenen nächsten Schritt versehen ist.

10. System nach Anspruch 7, wobei das Protokoll und die Zeitplanregion weiter ein Befolgen von Zeitbeschränkungen des klinischen Arbeitsflusses durch horizontales Ausrichten eines oder mehrere individueller Schritte des klinischen Arbeitsflusses mit einer zugehörigen Zeit in der Zeitplanregion markiert.

11. System nach Anspruch 10, wobei das Protokoll und die Zeitplanregion in Verbindung mit dem Zeitplan weiter eine Visualisierung einer Abweichung eines aktuellen Schritts von seiner Zeitvorgabe in dem klinischen Arbeitsfluss bereitstellt.

12. System nach Anspruch 7, wobei jede Zeile des Protokolls von klinischen Erkenntnissen, Ereignissen und Maßnahmen einen Hinweise auf ein Datum und eine Uhrzeit, eine Beschreibung, Ergebnisse und eine Identifikation einer Partei, welche ein entsprechendes eines von den klinischen Erkenntnissen, Ereignissen und Maßnahmen durchgeführt hat, bereitstellt, wobei weiter Einträge innerhalb des Protokolls von klinischen Erkenntnissen, Ereignissen und Maßnahmen intern mittels definierbarer Kategorien gekennzeichnet sind, um Filtern der Einträge unter Verwendung einer entsprechenden Registerkarte für eine gewünschte von den Kategorien, zu ermöglichen, und wobei die definierbaren Kategorien noch weiter eines oder mehrere, ausgewählt aus der Gruppe, bestehend aus Verlauf, physikalisch, medizinisch, Labor, Bilder EKGs und Aufträge beinhalten.

13. System nach Anspruch 7, wobei die Differenzialdiagnoseregion mögliche Diagnosen vertikal auflistet, wobei jede Diagnose durch ihren Namen und einen Balken mit einer Länge, welche eine aktuelle Wahrscheinlichkeit der entsprechenden Diagnose repräsentiert, identifiziert.

14. System nach Anspruch 13, wobei eine Diagnose weiter hierarchisch in einen Satz von Unterdiagnosen strukturiert werden kann, welche grafisch durch eines von Erweitern oder Reduzieren einer Diagnose in einer baumartigen Ansicht berücksichtigt werden.

15. System nach Anspruch 7, wobei Informationen für den vorgeschlagenen nächsten Schritt in einer oder mehreren Zeilen präsentiert werden, welche durch eine Beschreibung einer Maßnahme eines entsprechenden nächsten Schritts, einer Identifikation einer Partei, welche für die Durchführung der Maßnahme des entsprechenden nächsten Schritts verantwortlich ist, und ein Kontrollkästchenfeld gekennzeichnet sind, wobei ein Auftreten eines Häkchens in dem Kontrollkästchenfeld darauf hinweist, dass der entsprechende nächste Schritt abgeschlossen wurde.

16. System nach Anspruch 7, wobei abgeschlossene Schritte der Informationen für den vorgeschlagenen nächsten Schritt weiter auf eine Art modifiziert werden, um optisch auf den Abschluss desselben hinzuweisen.

17. System nach Anspruch 16, weiter eine Schaltfläche umfassend, welche als Reaktion darauf, dass sie ausgewählt wird, eine entsprechende Maßnahme des vorgeschlagenen nächsten Schritts automatisch ausführt.

## Revendications

1. Procédé mis en oeuvre par ordinateur de gestion de processus cliniques et de prise de décision comprenant :
la réception d'informations cliniques à partir d'un système d'information hospitalier (100), les informations cliniques incluant des informations pertinentes pour la gestion d'un problème clinique particulier d'un patient considéré ;
à plusieurs reprises :
(a) la détermination d'une vue d'ensemble d'actions cliniques en fonction des informations cliniques, les actions cliniques comprenant au moins l'une sélectionnée à partir du groupe comportant : des actions cliniques effectuées, des actions cliniques qui sont actuellement effectuées et des actions cliniques pas encore effectuées,
(b) le fait d'examiner si les actions cliniques adhèrent à un protocole prédéterminé, le protocole prédéterminé incluant
(i) une ou plusieurs actions cliniques idéales et
(ii) une synchronisation pour une survenance des une ou plusieurs actions cliniques idéales en fonction des informations cliniques et du problème clinique particulier, et
(c) la détermination d'informations d'étape suivante suggérée pour l'utilisation dans l'assistance pour des décisions diagnostiques et thérapeutiques à prendre par un ou plusieurs prestataires de soins conjointement à un traitement du problème clinique particulier, et
l'affichage des informations d'étape suivante suggérée par le biais d'une interface utilisateur graphique (200) qui comprend une pluralité de régions (5) d'affichage interactives, dans lequel la pluralité de régions d'affichage interactives comprend :
une région de diagnostic différentiel pour fournir une représentation de probabilités pour un ou plusieurs diagnostics différentiels en fonction d'informations cliniques actuellement disponibles, et
une région d'étape suivante suggérée pour fournir les informations d'étape suivante suggérée ayant été déterminées pour assister des décisions diagnostiques et thérapeutiques à prendre par un ou plusieurs prestataires de soins, dans lequel les informations d'étape suivante suggérée comprennent l'une sélectionnée à partir du groupe comportant (a) une action suivante suggérée obtenue à partir d'un état actuel d'exécution du processus clinique et d'informations cliniques actuellement disponibles, et (b) une action suivante suggérée découlant d'une interrogation d'un module de support de décision en connexion avec un diagnostic différentiel de la région de diagnostic différentiel, l'interrogation étant configurée pour trouver une ou plusieurs étapes diagnostiques appropriées pour faire une distinction entre de multiples diagnostics différentiels.

2. Procédé selon la revendication 1, dans lequel la réception d'informations cliniques inclut la réception à partir d'une ou plusieurs sources internes au système d'information hospitalier ; et dans lequel la réception à partir d'une ou plusieurs sources internes au système d'information hospitalier inclut la réception d'informations cliniques à partir de l'un sélectionné à partir du groupe comportant (a) un système de gestion de processus/directives, (b) un module de support de décision, (c) un système d'enregistrement de patient électronique et (d) un dispositif utilisateur de système.

3. Procédé selon la revendication 1, dans lequel la réception d'informations cliniques inclut la réception à partir d'une ou plusieurs sources internes au système d'information hospitalier ; et dans lequel la réception à partir d'une ou plusieurs sources externes au système d'information hospitalier inclut la réception d'informations cliniques à partir d'un dispositif distant.

4. Procédé selon la revendication 1, dans lequel les un ou plusieurs prestataires de soins incluent au moins l'un sélectionné à partir du groupe comportant un médecin, une infirmière, un professionnel de la santé et un aide-soignant.

5. Support lisible par ordinateur ayant un programme informatique stocké dessus, dans lequel ledit programme comprend un ensemble d'instructions qui, quand elles sont exécutées par un ordinateur, exécutent les étapes du procédé selon la revendication 1.

6. Système de gestion de processus cliniques et de prise de décision comprenant :
des moyens pour recevoir des informations cliniques à partir d'un système d'information hospitalier (100), les informations cliniques incluant des informations pertinentes pour la gestion d'un problème clinique particulier d'un patient considéré ;
des moyens pour, à plusieurs reprises :
(a) la détermination d'une vue d'ensemble d'actions cliniques en fonction des informations cliniques, les actions cliniques comprenant au moins l'une sélectionnée à partir du groupe comportant : des actions cliniques effectuées, des actions cliniques qui sont actuellement effectuées et des actions cliniques pas encore effectuées,
(b) le fait d'examiner si les actions cliniques adhèrent à un protocole prédéterminé, le protocole prédéterminé comprenant
(i) une ou plusieurs actions cliniques idéales et
(ii) une synchronisation pour une survenance des une ou plusieurs actions cliniques idéales en fonction des informations cliniques et du problème clinique particulier, et
(c) la détermination d'informations d'étape suivante suggérée pour l'utilisation dans l'assistance pour des décisions diagnostiques et thérapeutiques à prendre par un ou plusieurs prestataires de soins conjointement à un traitement du problème clinique particulier, et
des moyens pour afficher les informations d'étape suivante suggérée par le biais d'une interface utilisateur graphique (200) qui comprend une pluralité de régions d'affichage interactives (5), dans lequel la pluralité de régions d'affichage interactives comprend :
une région de diagnostic différentiel pour fournir une représentation de probabilités pour un ou plusieurs diagnostics différentiels en fonction d'informations cliniques actuellement disponibles, et
une région d'étape suivante suggérée pour fournir les informations d'étape suivante suggérée ayant été déterminées pour assister des décisions diagnostiques et thérapeutiques à prendre par un ou plusieurs prestataires de soins, dans lequel les informations d'étape suivante suggérée comprennent l'une sélectionnée à partir du groupe comprenant (a) une action suivante suggérée obtenue à partir d'un état actuel d'exécution du processus clique et d'informations cliniques actuellement disponibles, et (b) une action suivante suggérée découlant d'une interrogation d'un module de support de décision en connexion avec un diagnostic différentiel de la région de diagnostic différentiel, l'interrogation étant configurée pour trouver une ou plusieurs étapes diagnostiques appropriées pour faire une distinction entre de multiples diagnostics différentiels.

7. Système selon la revendication 6, dans lequel la pluralité de régions d'affichage interactives comprend en outre :
(i) une région de carte de protocole et de temps pour fournir une représentation graphique d'un processus clinique représentatif du protocole prédéterminé, dans lequel la région de carte de protocole et de temps est adaptée en outre pour fournir une représentation graphique d'une carte de temps de processus clinique, dans lequel la carte de temps comprend une corrélation entre une progression du processus clinique avec (a) un temps qui s'est écoulé depuis une apparition des symptômes du problème clinique particulier, et (b) un temps qui est résiduel dabs une période de temps pour un traitement du problème clinique particulier, et
(ii) une région d'observations cliniques pour fournir un journal d'observations, d'événements et d'actions cliniques pertinents pour le problème clinique particulier, dans lequel les observations, événements et actions cliniques sont basées sur les informations cliniques.

8. Système selon la revendication 7, dans lequel la pluralité de régions d'affichage interactives comprend en outre :
une région de vue d'ensemble de données de patient de niveau élevé pour fournir des informations sur le patient considéré, dans lequel les informations incluent le nom du patient, le genre, des informations à partir desquelles l'âge du patient peut être distingué et l'emplacement physique actuel du patient, et dans lequel la région de vue d'ensemble de données de patient de niveau élevé est en outre adaptée pour fournir une représentation graphique à barres d'un temps depuis l'apparition d'un événement donnant lieu au problème clinique particulier du patient considéré.

9. Système selon la revendication 7, dans lequel la pluralité de régions d'affichage interactives comprend en outre :
une région d'explication pour fournir une explication pour une étape suivante suggérée donnée, l'explication incluant une description d'un raisonnement qui sous-tend l'étape suivante suggérée donnée obtenue à partir d'un module de support de décision sous-jacent, dans lequel l'explication dans la région d'explication est fournie en réponse à une activation par le biais d'une touche explication, dans lequel la touche explication est fournie avec les informations d'étape suivante suggérée correspondantes dans la région d'étape suivante suggérée.

10. Système selon la revendication 7, dans lequel la région de carte de protocole et de temps met en outre évidence un respect de contraintes de temps du processus clinique en alignant horizontalement une ou plusieurs étapes individuelles du processus clinique avec un temps correspondant sur la carte de temps.

11. Système selon la revendication 10, dans lequel la région de carte de protocole et de temps fournit en outre, en relation avec la carte de temps, une visualisation d'une déviation d'une étape actuelle par rapport à sa synchronisation idéale dans le processus clinique.

12. Système selon la revendication 7, dans lequel chaque ligne du journal d'observations, d'événements et d'actions cliniques fournit une indication d'une date et d'une heure, une description, des résultats et une identification d'une partie qui a effectuée l'une correspondante des observations, événements et actions cliniques, dans lequel en outre des entrées dans le journal d'observations, d'événements et d'actions cliniques sont étiquetées en interne par des catégories définissables pour permettre le filtrage des entrées en utilisant un onglet correspondant pour l'une désirée des catégories, et dans lequel en outre encore les catégories définissables incluent un ou plusieurs sélectionnés à partir du groupe comprenant historique, physique, médical, laboratoire, images, ECG et ordres.

13. Système selon la revendication 7, dans lequel la région de diagnostic différentiel fournit une liste de diagnostics possibles verticalement, dans lequel chaque diagnostic est identifié par son nom et une barre ayant une longueur représentative d'une probabilité actuelle du diagnostique correspondant.

14. Système selon la revendication 13, dans lequel un diagnostic peut en outre être structuré hiérarchiquement dans un ensemble de sous-diagnostics qui sont comptabilités graphiquement par une parmi la décompression ou la compression d'un diagnostic d'une manière de type vue arborescente.

15. Système selon la revendication 7, dans lequel des informations d'étape suivante suggérée sont présentées sur une ou plusieurs lignes **caractérisées par** une description d'une action d'une étape suivante correspondante, d'une identification d'une partie responsable pour effectuer l'action de l'étape suivante correspondante et une case à cocher, dans lequel une apparition d'une coche dans la case à cocher indique que l'étape suivante correspondante a été achevée.

16. Système selon la revendication 7, dans lequel en outre des étapes achevées des informations d'étape suivante suggérée sont modifiées de manière à indiquer visuellement l'achèvement de celles-ci.

17. Système selon la revendication 7, comprenant en outre une touche qui, en réponse au fait qu'il est sélectionné, exécute automatiquement une action correspondante de l'étape suivante suggérée.
